# EUROPEAN PATENT APPLICATION

(11) **EP 1 683 475 A1**
(43) Date of publication of application: **26.07.2006**
(21) Application number: 06001223.4
(22) Date of filing: 20.01.2006
(51) Int. Cl.: A61B 3/032

(54) **Visual acuity chart displaying apparatus and optometry apparatus**

(30) Priority: 24.01.2005 JP 2005015072
(71) Applicant: Kabushiki Kaisha TOPCON, Tokyo 174-8580 (JP)
(72) Inventor: Ikezawa, Yukio, Tokyo 174-8580 (JP)
(74) Representative: Vossius & Partner

(57) **Abstract**

A visual acuity chart displaying apparatus, which is thin, has many indexes, including minutely small index. It includes a first index indicating mode for illuminating the film member having indexes from the rear lighting the liquid crystal display device, and a second mode for retreating the film member from the position located in front of the liquid crystal device and displaying the index pattern on the liquid crystal display device to project the index or index pattern on the eye to be examined, and a mode switching control unit of the CPU to switch between the first and second mode.

## Description

The present invention relates to a visual acuity chart displaying apparatus for indicating an optometry index(optotype) (hereinafter referred to as merely "index(es)") to an eye to be examined and an optometry apparatus for measuring visual acuity of the eye to be examined based on how an examinee to view the index.

In optometry measurement using indexes, the indexes are indicated to an eye to be examined by a visual acuity chart displaying apparatus or an optometry apparatus as described in JP 04-300518 A (claims, specification paragraph [0011], and Fig. 1), JP 06-245904 A (claims and specification paragraphs [0008] to [0011]), JP 06-142052 A (specification paragraph [0012] and Fig. 1), JP 06-254050 A (claims, specification paragraph [0012], and Fig. 1), or JP 11-235313 A (specification paragraph [0009] and Fig. 1). When an examinee makes a response to a question asking how he/she views each of the indicated indexes, a visual acuity value of the eye to be examined is determined based on the examinee's response.

In a visual acuity chart displaying apparatus described in JP 04-300518 A, a plurality of measurement index plates housed in a housing unit are selectively moved on an indication surface located at a predetermined distance from the eye to be examined. The measurement index plates are moved by a first moving unit for moving the housing unit in a forward and backward direction and a second moving unit for moving the measurement index plates in a lateral direction or an upward direction. A lamp for illuminating the measurement index plates and a reflective mirror having a concave surface are disposed in the rear of the measurement index plates moved to the indication surface.

In a visual acuity chart displaying apparatus described in JP 06-245904 A, a film sheet which an index is printed on or bonded to is coiled in advance to a sheet coiling shaft. In order to perform an examination, the film sheet is pulled out from the sheet coiling shaft so that a target index thereon is located in a predetermined position. The index located in the predetermined position is illuminated with an illumination lamp disposed in the rear of the film sheet, whereby the index is projected on the eye to be examined.

In an optometry apparatus described in JP 06-142052 A, an index is illuminated with light from a light source, whereby the index is projected on the eye to be examined.

In an optometry apparatus described in JP 06-254050 A, various Landolt rings for visual acuity values are displayed on a liquid crystal display plate based on a program for controlling an index indicating order. It is determined whether the examinee's response is right or wrong, to thereby display a result obtained by the examination. In the optometry apparatus, left, right, top, and bottom breaks of each of the Landolt rings are composed of different liquid crystal elements, so that a Landolt ring having a break in any one of the four directions is displayed. In an optometry apparatus described in JP 11-235313 A, indexes are displayed on a liquid crystal plate as in the case of JP 06-254050 A.

In the visual acuity chart displaying apparatuses described in JP 04-300518 A and JP 06-245904 A, the lamp for illuminating the indexes and the reflective mirror are disposed in the rear of the indexes, which hinders a reduction of the apparatus in thickness. Therefore, there is a problem in that an inconvenience occurs, for example, in the case where a set space for the apparatus is limited.

When the index is illuminated with light radially emitted from the lamp, there is the case where the indicated index has some unevenness in brightness and contrast because the flat index plate and the flat chart are not uniformly illuminated. In order to eliminate the unevenness of illumination, a structure may be applied in which a diffusing plate is disposed between the lamp and the index plate to diffuse light from the lamp, thereby illuminating the index plate or the like with the diffused light. However, there occurs a problem in that the thickness of the apparatus and a manufacturing cost thereof increase as the number of parts increases.

In the visual acuity chart displaying apparatuses described in JP 04-300518 A and JP 06-245904 A, the indexes which are printed on or bonded to the index plate or the sheet are used, so there is an advantage that a small index can be also minutely expressed. However, it is necessary to separately provide the respective indexes. Therefore, when a limitation on the size of the apparatus is taken into account, the types of usable indexes and the number of usable indexes are also limited accordingly. In particular, although a Landolt ring is one of most normally used indexes, a Landolt ring for a lower visual acuity value (for example, 0.1) requires a large area for display on the index plate or the like, so the number of index plates or the number of sheets on which other indexes are formed is limited. Thus, it is difficult to perform various examinations by a single visual acuity chart displaying apparatus. Because the indexes which are printed on or bonded to the index plate or the sheet are used, it is impossible to use variable indexes such as indexes for dynamic vision examination.

In the optometry apparatus described in JP 06-142052 A, the light source for index illumination is provided in the rear of the index, which makes it difficult to reduce the size (thickness) of the apparatus. Therefore, as in the cases of the visual acuity chart displaying apparatuses described in JP 04-300518 A and JP 06-245904 A, an inconvenience occurs, for example, in the case where the set space for the apparatus is limited.

In the structure in which the index is displayed on the liquid crystal plate as in the cases of in JP 06-254050 A and JP 11-235313 A, many different indexes are prepared for index display control software, which leads to an advantage that it is possible to use a number of different indexes incomparably larger than-those in a structure using the index plate or the like. In addition to this, the variable indexes such as the indexes for dynamic vision examination can also be used. For example, when a backlight unit is contained in the apparatus, it is easy to reduce the thickness of the apparatus. However, because of the limitation on the number of dots of the liquid crystal display plate, there is a problem in that a small index (for example, a Landolt ring for a higher visual acuity value of 2.0) cannot be minutely expressed unlike the structure using the index plate or the like.

An object of the present invention is to provide a visual acuity chart displaying apparatus and an optometry apparatus, each of which can be thinned and reduced in size.

In addition, another object of the present invention is to provide a visual acuity chart displaying apparatus and an optometry apparatus, each of which is capable of uniformly illuminating an index member such as an index plate or a sheet, on which indexes are formed, to indicate the indexes each of which has no unevenness in brightness and contrast.

Further, another object of the present invention is to provide a visual acuity chart displaying apparatus and an optometry apparatus, each of which is capable of using many different indexes and minutely expressing a very small index to indicate the index.

In order to attain the above-described objects, a visual acuity chart displaying apparatus according to a first aspect of the present invention includes: a flat panel display; an index member including at least one index for eye examination which is formed thereon; moving means for moving the index member; and control means for controlling the moving means to move the index member in front of the flat panel display and turning on the flat panel display to illuminate the index member from the rear, to project the index on an eye to be examined.

According to a second aspect of the present invention, the visual acuity chart displaying apparatus according to the first aspect of the invention further includes display control means for displaying an index pattern for eye examination on the flat panel display, and in the visual acuity chart displaying apparatus, the control means controls the moving means and the display control means to selectively switch between: a first index indicating mode for moving the index member in front of the flat panel display and turning on the flat panel display to illuminate the index member from the rear to project the index on the eye to be examined; and a second index indicating mode for removing the index member from the position located in front of the flat panel display and projecting the index pattern on the flat panel display to project the index pattern on the eye to be examined.

According to a third aspect of the present invention, in the visual acuity chart displaying apparatus according to the second aspect of the invention, when the index member on which a plurality of indexes are formed is located in front of the flat panel display in the first index indicating mode, the display control means lights only a region of a display screen of the flat panel display to selectively illuminate at least one of the plurality of indexes.

According to a fourth aspect of the present invention, in the visual acuity chart displaying apparatus according to the second aspect of the invention, the control means performs switching to a third index indicating mode for displaying an index pattern of another index on the flat panel display while the index member is moved in front of the flat panel display and illuminated from the rear to project the index.

According to a fifth aspect of the present invention, the visual acuity chart displaying apparatus according to any one of the second to fourth aspects of the invention further includes operating means for selecting one of the index and the index pattern which is projected on the eye to be examined, and in the visual acuity chart displaying apparatus, the control means performs index indicating mode switching in accordance with the selected one of the index and the index pattern.

An optometry apparatus according to a sixth aspect of the present invention includes: a flat panel display; an index member including an index for eye examination which is formed thereon; moving means for moving the index member; control means for controlling the moving means to move the index member in front of the flat panel display and turning on the flat panel display to illuminate the index member from the rear; an optical system for projecting the index of the index member illuminated from the rear on an eye to be examined; and operating means operated to input a response regarding the index projected on the eye to be examined.

According to a seventh aspect of the present invention, the optometry apparatus according to the sixth aspect of the invention further includes display control means for displaying an index pattern for eye examination on the flat panel display, and in optometry apparatus, the optical system projects the index pattern displayed on the flat panel display to the eye to be examined, the operating means is operated to input a response regarding the index pattern projected on the eye to be examined, and the control means controls the moving means and the display control means to selectively switch between: a first index indicating mode for moving the index member in front of the flat panel display and turning on the flat panel display to illuminate the index member from the rear to project the index on the eye to be examined; and a second index indicating mode for removing the index member from the position located in front of the flat panel display and displaying the index pattern on the flat panel display to project the index pattern on the eye to be examined.

According to the first aspect or the sixth aspect of the present invention, the index provided on the index member is subjected to backlight illumination using the flat panel display. Therefore, the apparatus can be thinned or reduced in size as compared with a conventional structure having a lamp and a reflective mirror.

According to the first aspect or the sixth aspect of the present invention, the planer flat panel display is used as a light source for index illumination, so the index member can be uniformly illuminated from the rear. Therefore, it is possible to project the index having no unevenness in brightness and contrast on the eye to be examined.

According to the second aspect or the seventh aspect of the present invention, the first index indicating mode for indicating the index provided on the index member and the second index indicating mode for indicating the index pattern using the flat panel display can be switched therebetween. Therefore; many different indexes can be displayed on the flat panel display. When a small index, which is hard to display on the flat panel display, is provided on the index member, the small index can be minutely expressed to project the index on the eye to be examined.

In the accompanying drawings:
Fig. 1 is a schematic perspective view showing an example of an external structure of a visual acuity chart displaying apparatus according to a first embodiment of the present invention when the apparatus is viewed from the front;
Fig. 2 is a schematic perspective view showing an example of an internal structure of the visual acuity chart displaying apparatus according to the first embodiment of the present invention when the apparatus is viewed from the front;
Fig. 3 is a schematic sectional view showing the internal structure of the visual acuity chart displaying apparatus according to the first embodiment of the present invention when the apparatus is viewed from the side;
Fig. 4 is a schematic view showing an example of an index film portion of the visual acuity chart displaying apparatus according to the first embodiment of the present invention;
Fig. 5 is a schematic view showing an example of a film member of the visual acuity chart displaying apparatus according to the first embodiment of the present invention;
Fig. 6 is a block diagram showing an example of a control system of the visual acuity chart displaying apparatus according to the first embodiment of the present invention;
Fig. 7 is a schematic view showing examples of an index provided on the film member of each of the visual acuity chart displaying apparatuses according to the first and second embodiments of the present invention;
Fig. 8 is a schematic view showing examples of the index provided on the film member of the visual acuity chart displaying apparatus according to the first embodiment of the present invention and examples of an index pattern displayed on a liquid crystal display device of the visual acuity chart displaying apparatus according to the second embodiment of the present invention;
Fig. 9 is a block diagram showing an example of a control system of the visual acuity chart displaying apparatus according to the second embodiment of the present invention;
Fig. 10 is a schematic perspective view showing an example of an external structure of an optometry apparatus according to a third embodiment of the present invention when the apparatus is viewed from the front;
Fig. 11 is a schematic view showing an example of an optical system of the optometry apparatus according to the third embodiment of the present invention;
Fig. 12 is a schematic view showing an example of the optical system of the optometry apparatus according to the third embodiment of the present invention;
Fig. 13 is a schematic view showing an example of the optical system of the optometry apparatus according to the third embodiment of the present invention;
Fig. 14 is a block diagram showing an example of a control system of the optometry apparatus according to the third embodiment of the present invention; and
Fig. 15 is a block diagram showing an example of a control system of an optometry apparatus according to a fourth embodiment of the present invention.

Structural examples of preferred embodiments of the present invention will be described in detail with reference to the accompanying drawings. Hereinafter, visual acuity chart displaying apparatuses according to first and second embodiments of the present invention and optometry apparatuses according to third and fourth embodiments of the present invention will be described.

In each of the visual acuity chart displaying apparatus according to the first embodiment and the optometry apparatus according to the third embodiment, a flat panel display is used as a light source for index illumination. In each of the visual acuity chart displaying apparatus according to the second embodiment and the optometry apparatus according to the fourth embodiment, an index formed on an index member and an index pattern displayed on the flat panel display can be switched therebetween according to how to use.

### <First Embodiment>

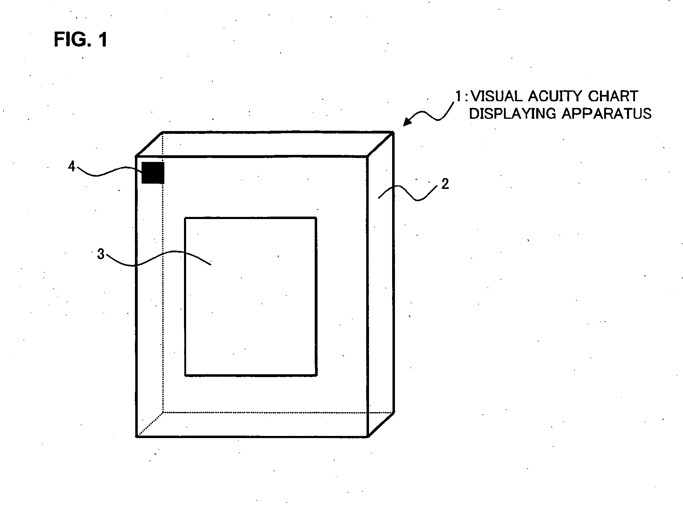

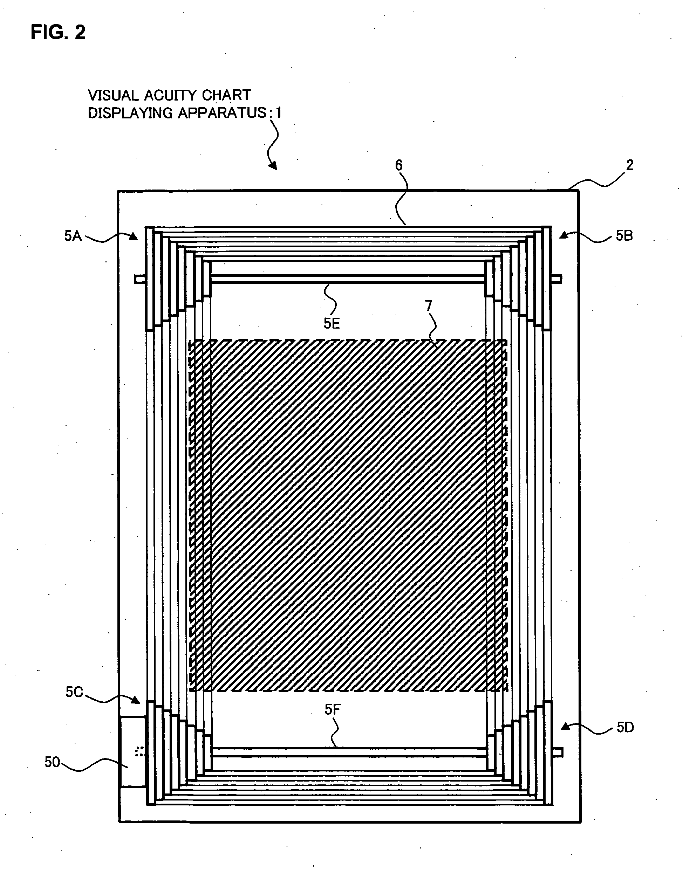

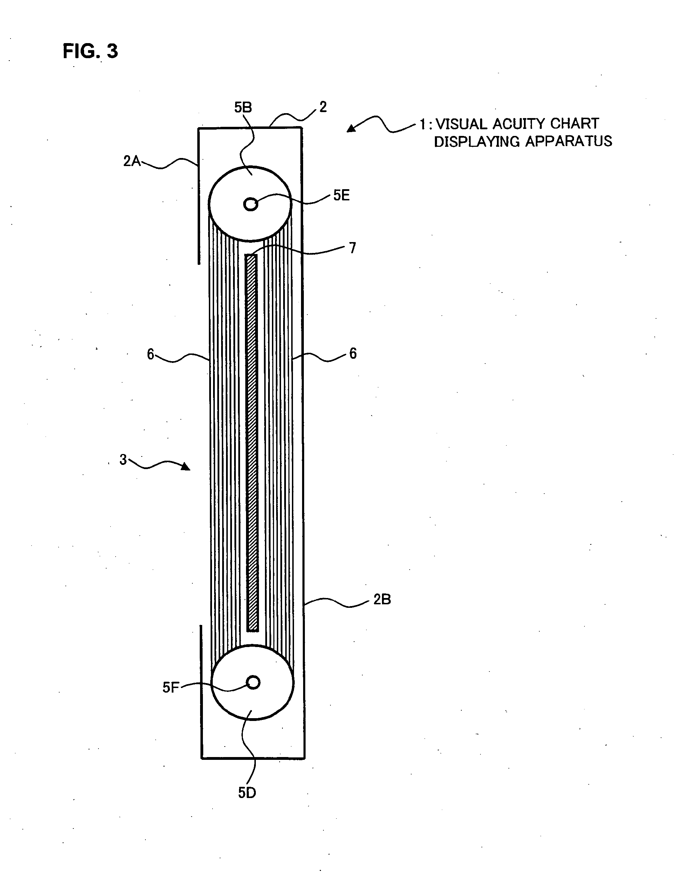

Figs. 1, 2, and 3 are schematic views showing the entire structure of the visual acuity chart displaying apparatus according to the first embodiment of the present invention. Fig. 1 is a schematic perspective view showing an external structure of the visual acuity chart displaying apparatus when the apparatus is viewed from the front. Fig. 2 is a schematic perspective view showing an internal structure of the visual acuity chart displaying apparatus when the apparatus is viewed from the front. Fig. 3 is a schematic sectional view showing the internal structure of the visual acuity chart displaying apparatus in section when the apparatus is viewed from the side.

A visual acuity chart displaying apparatus 1 according to this embodiment includes an apparatus box 2, an index display window 3 which is opened in the center of a front surface 2A of the apparatus box 2, and a receiver 4 for receiving an operating signal (such as an infrared signal) from a remote controller (described later).

The apparatus box 2 of the visual acuity chart displaying apparatus 1 includes roller portions 5A to 5D provided above and below the index display window 3, index film portions 6 on which optometry indexes are provided, and a liquid crystal display device 7 as a flat panel display provided in the rear of the index display window 3. A CPU for controlling the operation of each of parts of the apparatus and a ROM for storing various computer program, various control data, and the like (described later) are provided inside, for example, a rear surface 2B of the apparatus box 2.

The upper roller portions 5A and 5B are located to be freely rotatable about a rotational shaft 5E. The lower roller portions 5C and 5D are rotated in conjunction with a rotational shaft 5F rotated by a motor such as a stepping motor (described later) provided in a drive mechanism portion 50.

The index film portions 6 are circularly formed and wound around the upper roller portions 5A and 5B and the lower roller portions 5C and 5D so as to go therebetween. The index film portions 6 is driven in conjunction with the roller portions 5C and 5D rotated by the drive mechanism portion 50 and moved between the upper roller portions 5A and 5B which are freely rotated and the roller portions 5C and 5D in the vertical direction.

As shown in Fig. 2, the plurality of (for example, eight) index film portions 6 are provided. Each of the roller portions 5A to 5D includes a plurality of (for example, eight) rollers for supporting the respective index film portions 6. These plurality of rollers are arranged such that every roller diameter gradually becoming shortened in the direction from the outside of the apparatus to the inside thereof.

As shown in Figs. 2 and 3, the liquid crystal display device 7 provided in the rear of the index display window 3 to be arranged in a space inside the index film portions 6 which are circularly formed.

The drive mechanism portion 50 includes a clutch mechanism (described later) for selectively transmitting the torque of the stepping motor to a set of rollers out of the plurality of rollers included in the roller portions 5C and 5D. The plurality of index film portions 6 can be selectively moved up and down by the clutch mechanism.

In this embodiment, although the rotational shafts 5E and 5F for the roller portions 5A to 5D are arranged in the lateral direction, the rotational shafts may be arranged in the vertical direction to move the index film portions 6 in the lateral direction. Both the rotational shafts arranged both in the lateral direction and in the vertical direction may be provided so as to use the index film portions moved vertically and laterally by overlapping them.

Although not shown, the liquid crystal display device 7 includes a liquid crystal panel composed of a glass substrate, a liquid crystal panel composed of transparent electrode, a backlight illumination source for illuminating the liquid crystal panel from the rear, and a liquid crystal driving substrate. For example, a color display device capable of emitting white illumination light is used as the liquid crystal display device 7. The liquid crystal display device 7 is an example of a "flat panel display" in the present invention.

In addition to the liquid crystal display device 7, it is possible to use, as the flat panel display in the present invention, an arbitrary display device whose box has a plate shape and display surface is flat, such as a plasma display panel (PDP), an organic electro-luminescence (EL) display, an inorganic EL display, a field emission display (FED) (such as a surface-conduction electron-emitter display (SED)), a light emitting diode (LED), or a vacuum fluorescent display (VFD).

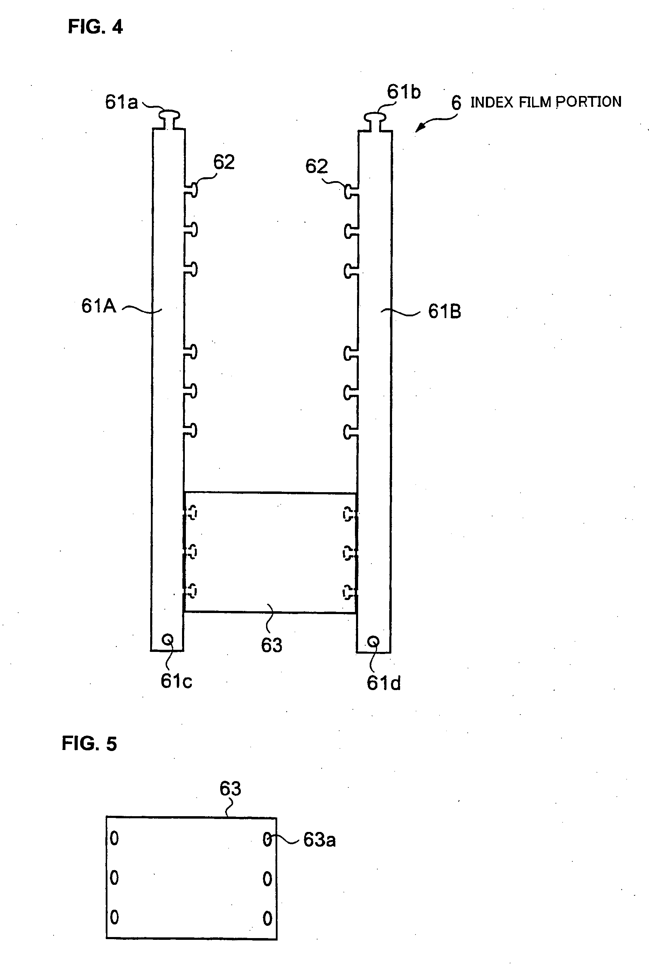

Figs. 4 and 5 show an example of each of the plurality of index film portions 6. Each of the index film portions 6 includes a set of right and left guide belts 6 1 A and 61B and a film member 63 composed of, for example, a white film which an optometry index is printed on or bonded to. The film member 63 is an example of an "index member" in the present invention.

An interval at which the right and left guide belts 61A and 61B are provided is changed according to an interval between the rollers of each of roller portions 5A to 5D and a width of the film member 63 is designed based on the interval between the rollers. That is, the guide belts 61A and 61B wound around outside rollers of the plurality of rollers of roller portions 5A to 5D are located at a wider interval and the film member 63 attached to the guide belts 61A and 61B has a wider width. On the other hand, the guide belts 61A and 61B wound around inside rollers of the plurality of rollers of roller portions 5A to 5D are located at a narrower interval and the film member 63 attached to the guide belts 61A and 61B has a narrower width.

A plurality of film hooks 62, each of which has substantially a T-shape, are provided on the insides (opposed sides) of the guide belts 61A and 61B. A plurality of hole portions 63a are formed near right and left end portions of the film member 63 at a predetermined interval. The film hooks 62 are inserted into the hole portions 63a to attach the film member 63 to the guide belts 61A and 61B.

The number of film members 63 attached to the guide belts 61 A and 61B is arbitrary. When the plurality of film members 63 are to be attached to the set of guide belts 61A and 61 B, it is desirable to prevent the film members 63 from overlapping with one another.

A belt hook 61a having substantially a T-shape is formed in one end of the guide belt 61A and a hole portion 61c is formed in the other end thereof. The belt hooks 61 a is inserted into the hole portion 6 1 c to connect the one end of the guide belt 6 1 A to the other end thereof, thereby circularly forming the guide belt 61A. Similarly, a belt hook 61b having substantially a T-shape is formed in one end of the guide belt 61B and a hole portion 61d is formed in the other end thereof. The belt hooks 61b is inserted into the hole portion 61d to connect the one end of the guide belt 61B to the other end thereof, thereby circularly forming the guide belt 61B.

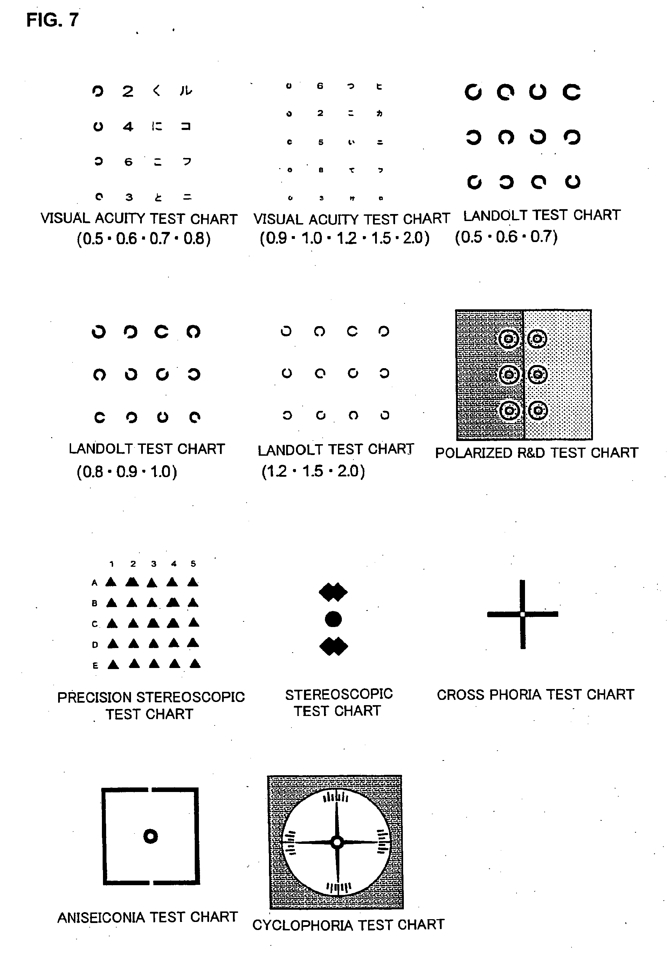

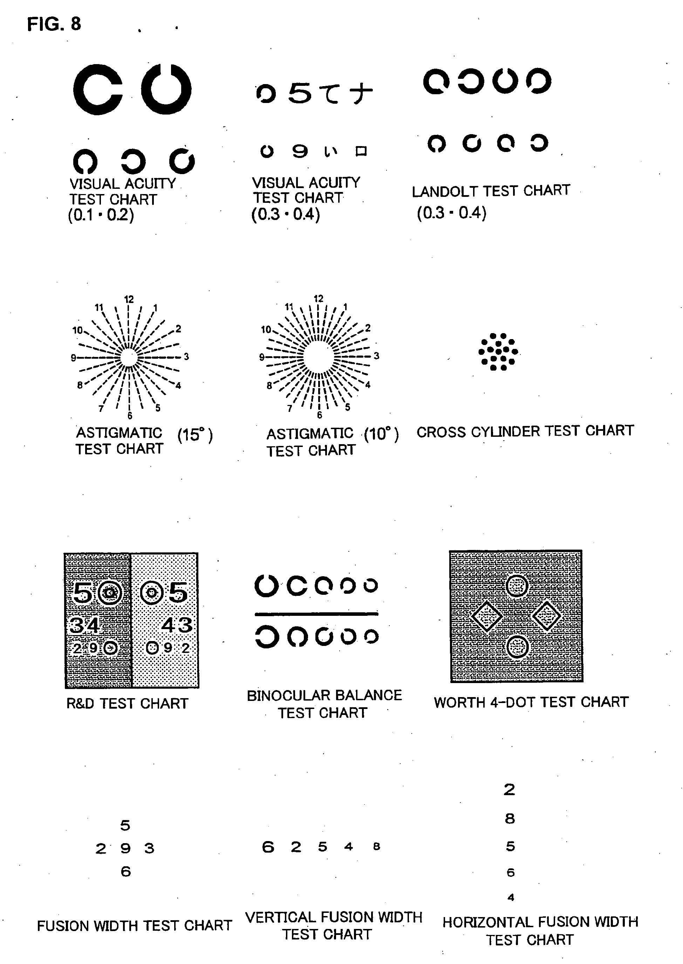

The index (chart) provided on the film member 63 includes at least one of a plurality of indexes shown in, for example, Figs. 7 and 8. Fig. 7 shows visual acuity test charts and Landolt test charts which are for visual acuity values equal to or larger than a predetermined value (for example, 0.5), a polarized red-and-green (R&D) test chart, a precision stereoscopic test chart, a stereoscopic test chart, a cross phoria test chart, an aniseiconia test chart, and a cyclophoria test chart. These charts are particularly minute indexes, each of which has a small size, indexes using polarization functions during an examination, or the like. Fig. 8 shows visual acuity test charts and a Landolt test chart which are for visual acuity values equal to or smaller than a predetermined value (for example, 0.4), astigmatic test charts (15° and 10°), a cross cylinder test chart, a red-and-green (R&D) test chart, a binocular balance test chart, a Worth 4-dot test chart, a fusion width test chart, a vertical fusion width test chart, and a horizontal fusion width test chart. Although not shown, it is also possible to indicate a color blindness test chart or a contrast visual acuity test chart. In addition, for example, a dynamic visual acuity test chart or a depth perception test chart (three-rod meter), which is displayed as a moving picture, can be indicated. An arbitrary index other than the above-mentioned indexes can be suitably provided on the film member 63 to use the index for the examination.

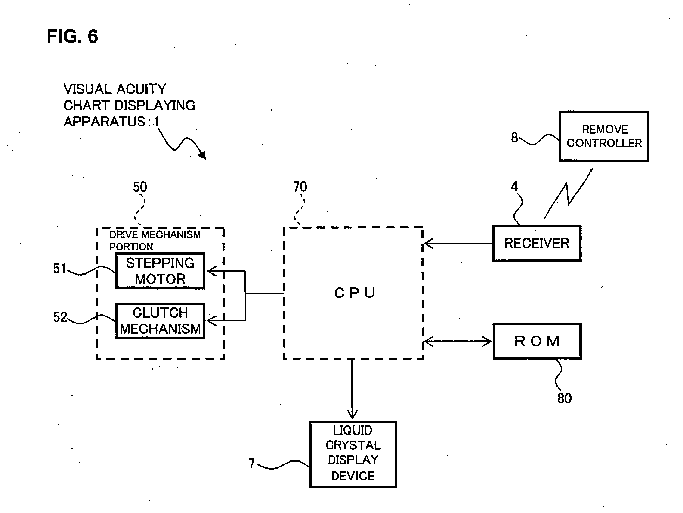

Fig. 6 shows an example of a control system of the visual acuity chart displaying apparatus 1. The operation of each of parts of the visual acuity chart displaying apparatus 1 is controlled by a CPU 70. The CPU 70 corresponds to "control means" in the present invention and executes the control processing of each of the parts of the apparatus based on control programs and control data which are stored in a ROM 80.

The remote controller 8 includes buttons and switches which are used for remotely performing a selection input operation of an index projected on an eye to be examined, an ON/OFF operation of a power source of the visual acuity chart displaying apparatus 1, an operation of turning on/off a backlight unit, and the like. Upon receiving an operation from an operator, the remote controller 8 transmits an operation signal corresponding to the contents the received operation to the receiver 4. When the receiver 4 receives the operation signal, the visual acuity chart displaying apparatus 1 executes an operation corresponding to the operation signal.

The remove controller 8 is an example of "operating means" in the present invention. In addition to such a radio remote control device, a cable remote control device, a device (operational panel) formed on the box 2 of the visual acuity chart displaying apparatus 1, or the like, can be used as the operating means in the present invention. The operating means is not limited to a dedicated device for operating the visual acuity chart displaying apparatus 1, and thus may be a controller capable of also operating other ophthalmologic apparatuses, an input device such as a keyboard or a mouse in a computer apparatus, or the like.

The drive mechanism portion 50 is an example of "moving means" in the present invention, for moving the film member (index member) 63, on which the index is provided, to set the film member 63 in a predetermined index indicating position (described later), and includes a stepping motor 51 and a clutch mechanism 52.

In the present invention, the index member and the moving means are not limited to those described in this embodiment. In other words, an arbitrary index member on which an optometry index is formed can be employed as the index member in the present invention. An arbitrary moving means capable of moving the index member to set the index member in the position in which the index display window 3 is located and removing the index member from the position in which the index display window 3 is located can be employed as the moving means in the present invention.

The ROM 80 stores information for specifying the index formed on each of the film members 63 of the plurality of index film portions 6. The information is, for example, an association table in which index specifying information for specifying an index, such as an index name (see Figs. 7 and 8), is associated with film specifying information for specifying the film member 63 on which the index is formed.

An example of the association table will be described. Assume that the number of index film portions 6 is eight (see Fig. 2) and the two film members 63 are attached to each of the index film portions 6. In addition, assume that one of the two film members 63 of each of the index film portions 6 is set in the position in which the upper roller portions 5A and 5B are located and the other thereof is set in the position in which the lower roller portions 5C and 5D are located. For example, assume that the index "cross cylinder test chart" is set in a position in which the third rollers of the upper roller portions 5A and 5B which are counted from the outside are located. In such a case, film specifying information for "cross cylinder test chart" is defined as a "upper side 3". Then, the index specifying information "cross cylinder test chart" is associated with the film specifying information "upper side 3". In this way, the index specifying information for specifying the index formed on each of the film members 63 is associated with any one of the film specifying information "upper side 1" to "upper side 8" and "lower side 1" to "lower side 8", to thereby produce the above-mentioned association table.

When the film member 63 of the index film portion 6 is exchanged for another film member to use another index, the contents of the association table can be rewritten by the remote controller 8, the computer apparatus connected to the visual acuity chart displaying apparatus 1, or the like.

According to the visual acuity chart displaying apparatus 1 having the above-mentioned structure, the following index indicating processing can be performed.

When the operator selected an index by operating the remote controller 8, the CPU 70 determines the film member 63 of the index film portion 6 on which a target index is formed by consulting the association table.

Next, the CPU 70 controls the clutch mechanism 52 of the drive mechanism portion 50 to specify rollers for driving the guide belts 61A and 61B to which the film member 63 having the target index are attached. Then, the CPU 70 controls the clutch mechanism 52 so as to transfer the torque of the stepping motor 51 to the specified rollers.

The stepping motor 51 is rotated by a predetermined rotational amount to rotate the specified rollers, thereby setting the film member 63 on which the target index is formed in the position in which the index display window 3 is located (index indicating position). Note that the predetermined rotational amount is determined in advance based on a distance between the set position of the target index (roller position) and the index indicating position.

Then, the CPU 70 turns on the liquid crystal display device 7 to illuminate the target index set in the index indicating position from the rear. Therefore, the target index is projected on the eye to be examined of an examinee, which is opposed to the index indicating position at a predetermined distance (for example 5, meters).

According to the visual acuity chart displaying apparatus 1 in this embodiment, the flat panel display (liquid crystal display device 7) is used as the light source for emitting illumination light for index illumination. Therefore, the apparatus can be thinned as compared with the conventional case where the lamp and the mirror are used.

The index member is illuminated from the rear by the flat panel display having a flat light emitting surface. Therefore, the index member can be uniformly illuminated, so that it is possible to project the index having no unevenness in brightness and contrast.

### <Second Embodiment>

Next, a visual acuity chart displaying apparatus according to another embodiment of the present invention will be described. The visual acuity chart displaying apparatus according to this embodiment has, for example, the same structure as that in the first embodiment except the control system. In the following description, the same reference numerals are provided for the same constituent parts as those in the first embodiment. The contents described in the first embodiment can be arbitrary applied to this embodiment.

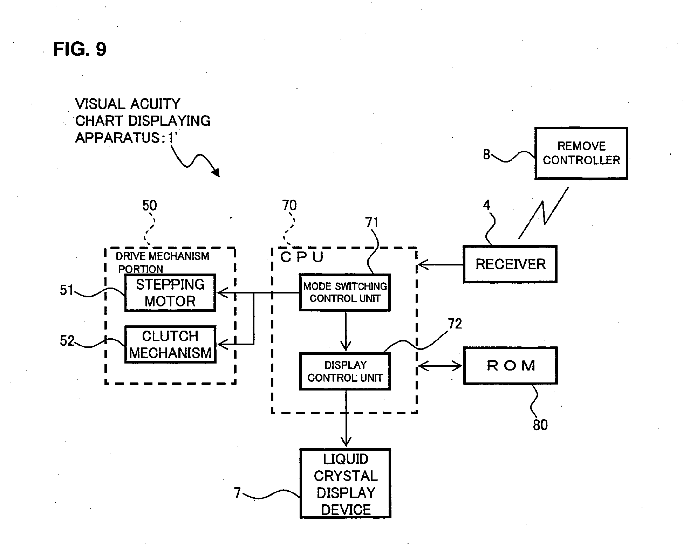

Fig. 9 shows an example of a control system of a visual acuity chart displaying apparatus 1' according to this embodiment. The CPU 70 includes a mode switching control unit 71 (control means) for switching between operational modes for projecting the index on the eye to be examined (not shown) (index indicating modes) and a display control unit 72 for controlling the liquid crystal display device 7.

The index indicating modes used for the visual acuity chart displaying apparatus 1' include a first index indicating mode and a second index indicating mode. The first index indicating mode is a mode for projecting the index of the film member 63 on the eye to be examined. The second index indicating mode is a mode for projecting the index (pattern) on the eye to be examined by displaying the index pattern on the liquid crystal display device 7. Although there is further a third index indicating mode, it will be described later in a modified example. Image data for each index pattern indicated in the second index indicating mode is stored in the ROM 80.

It is desirable to selectively employ, for example, a small index as shown in Fig. 7 as the index indicated in the first index indicating mode. It is desirable to selectively employ, for example, a large-size index or a variable index with movement as shown in Fig. 8 as the index indicated in the second index indicating mode. That is, the small index can be minutely expressed by forming the index on the film member 63 by printing or the like. With respect to the large-size index, images data is stored in the ROM 80, so that many different index patterns can be used and the index which is to be provided on the film member 63 can be carefully selected therefrom. The variable index cannot be indicated using the film member 63 but can be indicated by the liquid crystal display device 7.

The first index indicating mode is executed according to, for example, the following three steps by the mode switching control unit 71.
(1-1) The film member 63 of the index film portion 6 on which a target index is formed is determined with reference to the association table described in the first embodiment. Next, the clutch mechanism 52 of the drive mechanism portion 50 is controlled to specify rollers for driving the guide belts 61A and 61B to which the film member 63 having the target index are attached. Then, the clutch mechanism 52 is controlled so as to transmit the torque of the stepping motor 51 to the specified rollers. Note that the target index is, for example, an index selectively inputted from the remote controller 8 by the operator.
(1-2) The stepping motor 51 is rotated by a predetermined rotational amount to rotate the rollers specified in (1-1), thereby moving the target index in the position in which the index display window 3 is located (index indicating position). Note that the predetermined rotational amount is determined in advance based on a distance between the set position of the target index (roller position) and the index indicating position.
(1-3) The display control unit 72 is controlled to turn on the liquid crystal display device 7, thereby illuminating the target index set in the index indicating position in (1-2).
   According to (1-1) to (1-3), the target index is set in the position in which the index display window 3 is located and illuminated from the rear by the liquid crystal display device 7. Therefore, the target index is projected on the eye to be examined.
   The second index indicating mode is executed according to, for example, the following two steps.
(2-1) The clutch mechanism 52 and the stepping motor 51 are controlled to retreat the film member 63 set in the position in which the index display window 3 is located to the position in which the upper roller portions 5A and 5B or the lower roller portions 5C and 5D are located.
(2-2) Image data (moving picture data) for a target index pattern is obtained from the ROM 80 and the display control unit 72 is controlled to display the index pattern expressed by the image data on the liquid crystal display device 7.

According to (2-1) and (2-2), the target index pattern displayed on the liquid crystal display device 7 to project the index on the eye to be examined through the index display window 3.

According to the visual acuity chart displaying apparatus 1' having the above-mentioned structure, as in the first embodiment, the apparatus can be thinned and the index having no unevenness in brightness and contrast can be projected on the eye to be examined.

The first index indicating mode for indicating the index provided on the film member 63 and the second index indicating mode for indicating the index using the liquid crystal display device 7 can be switched therebetween. Therefore, when image data for many different indexes (index patterns) are stored in the ROM 80, the many different indexes can be displayed on the liquid crystal display device 7 to project the indexes on the eye to be examined. When a small index hard to display on the liquid crystal display device 7 is provided on the film member 63, it is possible to minutely express the small index. Therefore, the number of types of performable examinations and an examination range can be increased and the examination precision is improved.

### (Modified Examples)

A modified example of the visual acuity chart displaying apparatus 1' according to this embodiment will be described. When the plurality of indexes such as the visual acuity test charts and the Landolt test charts as shown in Fig. 7 are set in the position in which the index display window 3 is located in the first index indicating mode, the control is made such that only a region of the liquid crystal display device 7 is lighted. Therefore, of the plurality of indexes, one index or two or more indexes can be selectively illuminated to project the indexes on the eye to be examined. A lighting region of the liquid crystal display device 7 is changed by the display control unit 72 in response to, for example, an index selection input from the remote controller 8. For example, when the Landolt test chart having twelve Landolt rings provided thereon as shown in Fig. 7 is used, the Landolt ring indicated to the eye to be examined can be changed using cursor keys (not shown) of the remote controller 8. Therefore, the examinee can easily recognize a target index of the plurality of indexes indicated. This modified example corresponds to an index mask function (for example, see JP 08-215146 A).

In another modified example, it is possible to apply the third index indicating mode using a combination of the index provided on the film member 63 and the index pattern displayed on the liquid crystal display device 7. The index suitable for the third index indicating mode includes an index whose part is stationary and remaining part is moving, such as a three-rod meter. A stationary part of such an index is provided on the film member 63 a moving part thereof is displayed on the liquid crystal display device 7 located in the rear of the film member 63. At this time, the display control is made such that a screen region of the liquid crystal display device 7 which corresponds to the stationary part of the index which is provided on the film member 63 is lighted with, for example, white illumination light.

### <Third Embodiment>

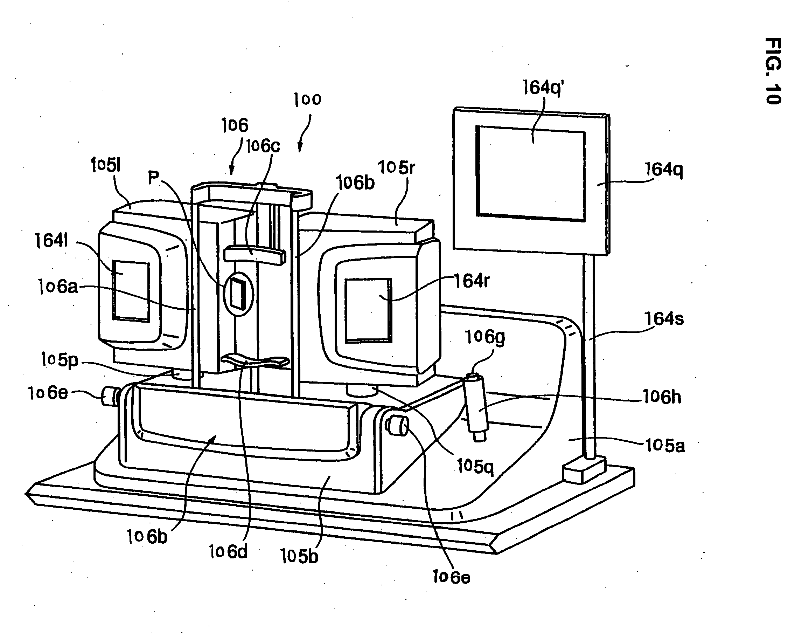

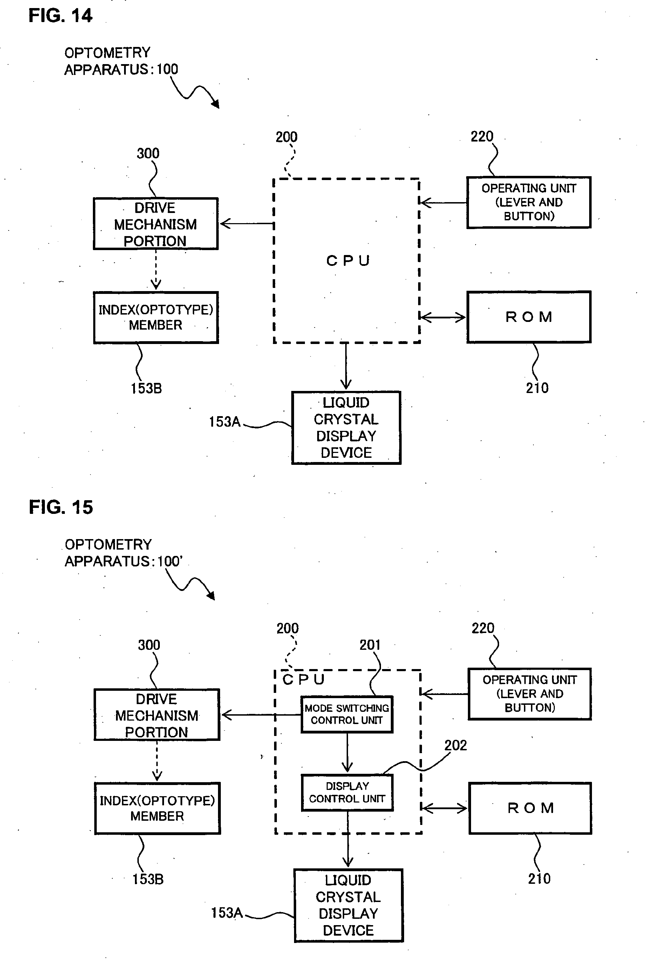

An optometry apparatus according to a third embodiment of the present invention will be described. Figs. 10 to 13 show an external structure of the optometry apparatus according to this embodiment and an optical system thereof and Fig. 14 shows a control system thereof.

As shown in Fig. 10, an optometry apparatus 100 to be used is placed on an optometry table whose height is adjustable (not shown). The examinee performs an examination in an optometry chair provided together with the optometry table.

A pillar 164s is erected on the optometry table. A liquid crystal monitor 164q is provided in an upper end of the pillar 164s. The liquid crystal monitor 164q may be not provided.

The optometry apparatus 100 includes a base portion 105a, a drive mechanism box 105b provided on the base portion 105a, a set of left and right optical head sections 1051 and 105r housing measurement optical systems described later, and a face holding device 106 for holding the face of the examinee during an examination.

The optical head sections 1051 and 105r are supported on pillars 105p and 105q and separately driven three-dimensionally by the drive mechanism box 105b. A liquid crystal monitor 1641 is provided on a front surface of the optical head section 105l and a liquid crystal monitor 164r is provided on a front surface of the optical head section 105r. An anterior segment image of the eye to be examined, an eye fundus reflection image thereof, and the like, which are obtained during the examination are displayed on each of the liquid crystal monitors 1641 and 164r. An examiner or an assistant can recognize whether or not the examinee properly performs the examination based on the displayed anterior segment image.

The face holding device 106 includes a set of left and right pillars 106a and 106b, a forehead support 106c supported by a member connecting between upper ends of the pillars 106a and 106b, and a chin rest 106d provided under the forehead support 106c. The forehead support 106c is a member which is in contact with the forehead of the examinee during the examination. The forehead support 106c is formed in an arc shape to improve the contact with the forehead and the position thereof is adjustable forward and backward. The chin rest 106d is a member on which the examinee rests the chin during the examination and the position thereof is adjustable in vertical directions by a set of right and left knobs 106e. In order to perform the examination, the examinee rests the chin on the chin rest 106d and brings the forehead into contact with the forehead support 106c and hold the face still to stand by for examination.

The drive mechanism box 105b includes XYZ drive mechanisms for separately driving the pillars 105p and 105q three-dimensionally. Although the details on the XYZ drive mechanisms are not shown, a known structure using, for example, a pulse motor and a feed screw can be employed for each of the mechanisms. Therefore, the pillars 105p and 105q, that is, the optical head sections 1051 and 105r are separately driven three-dimensionally.

The drive mechanism box 105b further includes a rotation drive mechanism for separately rotating the pillars 105p and 105q horizontally. For example, a structure in which a pulse motor and gears for transmitting the torque of a pulse motor to the respective pillars 105p and 105q are combined can be employed as the rotation drive mechanism. The rotation drive mechanism is constructed so as to rotate the pillars 105p and 105q, that is, the optical head sections 1051 and 105r reversely to each other about eyeball rotational points of the right and left eyes of the examinee.

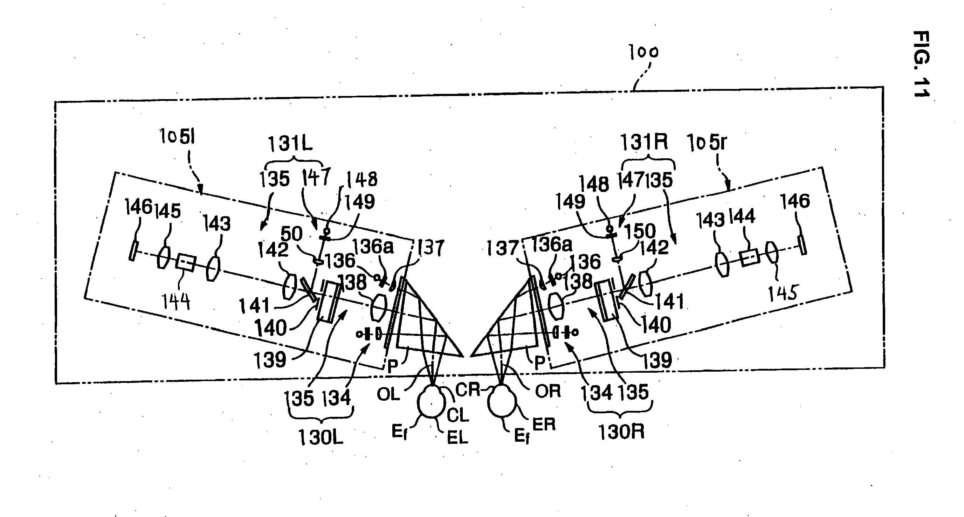

Each of the optical head sections 1051 and 105r houses various optical systems shown in Figs. 11 to 13 as described later. In the optical head sections 1051 and 105r, the optical systems are operated to perform objective refraction measurement and subjective refraction measurement on both eyes of the examinee.

A joystick lever (hereinafter may be merely referred to a lever) 106h is provided on the base portion 105a. The lever 106h can be tilted, for example, in eight directions such as the upward direction, the downward direction, the right direction, the left direction, the upper right direction, the upper left direction, the lower right direction, and the lower left direction. The lever 106h may be tilted in four directions such as the upward direction, the downward direction, the right direction, and the left direction. A button 106g is provided on an upper portion of the lever 106h. The examinee suitably operates the lever 106h and the button 106g to perform the examination.

Measurement optical systems for measuring refractive powers of the right and left eyes of the examinee, which are housed in the optical head portions 1051 and 105r will be described in detail. As shown in Figs. 11 to 13, a measurement optical system of the optical head section 1051 for measuring the left eye of the examinee includes an anterior segment image taking optical system 130L, an XY-alignment optical system 131L, an index projecting optical system 132L, and a refractive power measuring optical system 133L. A measurement optical system of the optical head section 105r for measuring the right eye of the examinee has the same structure as that in the measurement optical system of the optical head section 1051. Hereinafter, the measurement optical system of the optical head section 1051 for left eye measurement will be described unless otherwise specified.

The anterior segment image taking optical system 130L provided in the optical head section 1051 includes an anterior segment illuminating optical system 134 and an image taking optical system 135.

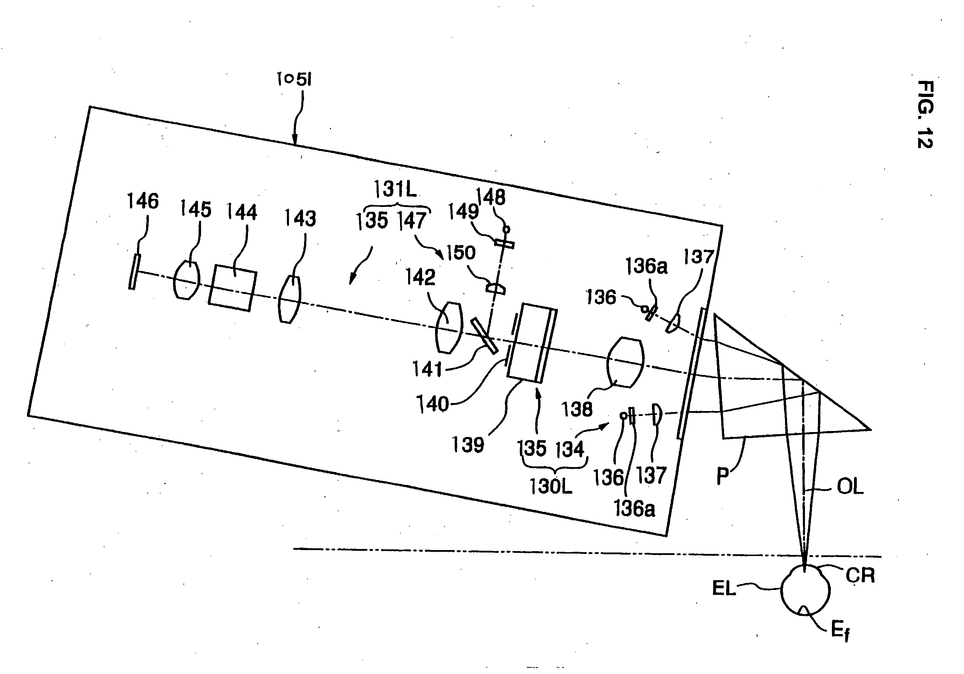

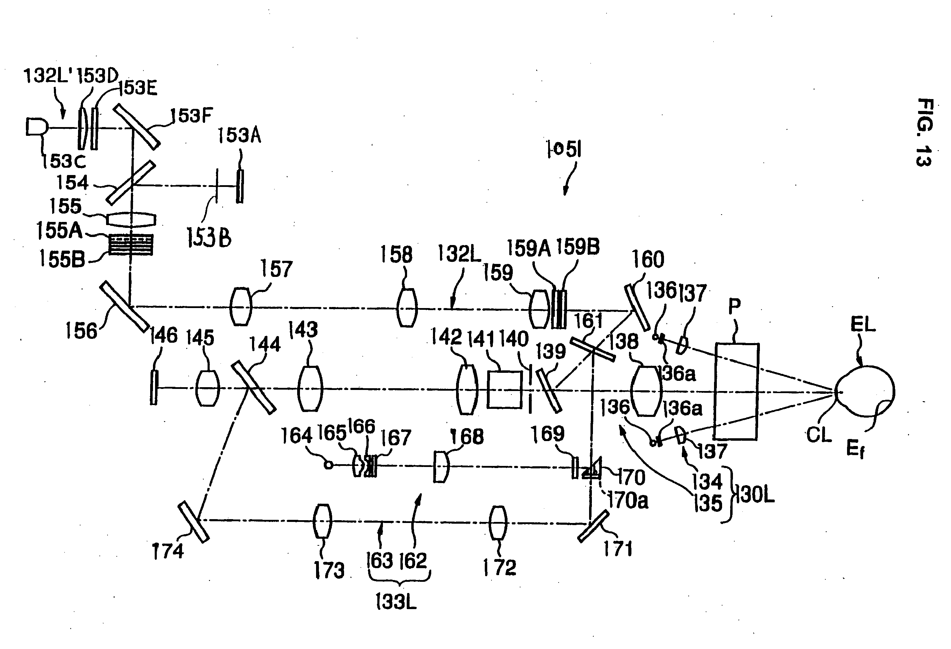

As shown in Figs. 12 and 13, the anterior segment illuminating optical system 134 includes light sources 136 for illuminating the anterior segment of the left eye of the examinee (eye to be examined EL), diaphragms 136a for limiting cross sectional areas of light fluxes emitted from the light sources 136, and projection lenses 137 for projecting the light fluxes passing through the diaphragms 136a to the anterior segment of the eye to be examined EL.

The image taking optical system 135 includes a prism P, an objective lens 138, a dichroic mirror 139, a diaphragm 140, a dichroic mirror 141, relay lenses 142 and 143, a dichroic mirror 144, a CCD 146, and a CCD lens 145 for imaging a light flux on a light receiving surface of the CCD 146. Reflection light on the anterior segment of the eye to be examined EL which is illuminated by the anterior segment illumination optical system 134 is incident on the prism P. A light flux reflected on a reflective surface of the prism P is incident on the objective lens 138.

The XY-alignment optical system 131L is an optical system for aligning the measurement optical system of the optical head section 1051 with the eye to be examined EL in X- and Y-directions. The XY-alignment optical system 131L includes an alignment illumination optical system 147 for projecting an alignment light flux on the eye to be examined EL and the image taking optical system 135 for receiving reflection light on the eye to be examined EL, serving as an alignment light receiving optical system. Assume that the lateral direction as viewed from the examinee is an X-direction and the vertical direction as viewed from the examinee is a Y-direction. In addition, assume that the depth direction of the optometry apparatus 100 as viewed from the examinee is a Z-direction.

As shown in Figs. 11 and 12, the alignment illumination optical system 147 includes an illumination light source 148 for emitting a light flux for XY-directional alignment, a diaphragm 149 for alignment index, a relay lens 150, the dichroic mirror 141, the diaphragm 140, the dichroic mirror 139, the objective lens 138, and the prism P.

The index projecting optical system 132L includes a liquid crystal display device 153A for emitting light for index illumination, index members 153B on which various indexes are formed, a half mirror 154 for reflecting the light from the liquid crystal display device 153A, a collimator lens 155, rotary prisms 155A and 155B for adjusting prism power and a prism base direction in a phoria examination, and a reflective mirror 156. The index projecting optical system 132L further includes a movable lens 157 used to perform, for example, fixation and fogging on the eye to be examined EL, relay lenses 158 and 159, variable cross cylinder lenses (VCC lenses) 159A and 159B for adjusting astigmatic power and an astigmatic axial angle in an astigmatic examination, a reflective mirror 160, a dichroic mirror 161, the dichroic mirror 139, the objective lens 138, and the prism P.

The indexes shown in, for example, Figs. 7 and 8 are formed on the index members 153B. Each of the index members 153B is the film member as described in the first embodiment, a turret plate, or the like. When the index film member is to be used, the plurality of index members 153B are provided therein. The respective index members 153B are selectively moved by the drive mechanism portion to insert or remove the index members into or from the optical path. When the turret plate is to be used, one or plural index members 153B are provided thereon. The turret plate is rotated by a drive mechanism portion to insert or remove the respective index members 153B into or from the optical path.

The rotary prisms 155A and 155B are separately rotated by a pulse motor or the like. When the rotary prisms 155A and 155B rotate in directions reverse to each other, the prism power continuously changes. On the other hand, when the rotary prisms 155A and 155B integrally rotate in the same direction, the prism base direction continuously changes.

The VCC lens 159A has a convex surface and the VCC lens 159B has a concave surface. The VCC lenses 159A and 159B are separately rotated by a pulse motor or the like. When the VCC lenses 159A and 159B rotate in directions reverse to each other, astigmatic power changes. On the other hand, when the VCC lenses 159A and 159B integrally rotate in the same direction, an astigmatic axial angle changes.

The movable lens 157 is moved in the optical axis direction by a pulse motor or the like to change spherical power added to the eye to be examined EL. When the movable lens 157 is moved in the optical axis direction by a distance corresponding to the refractive power of the eye to be examined EL by the operator, the fixation and fogging can be performed on the eye to be examined EL.

A fusion index projecting optical system 132L' is provided in a light passing through direction of the half mirror 154 of the index projecting optical system 132L. The fusion index projecting optical system 132L' includes an LED 153C for emitting illumination light, a collimator lens 153D, a fusion frame chart 153E, and a total reflection mirror 153F. The fusion frame chart 153E has a square light transmission window (fusion window) and a light shielding portion which are formed therein (not shown). The collimator lens 153D has a diffusing surface for uniformly illuminate the fusion frame chart 153E with light from the LED 153C. The fusion frame chart 153E is indicated if necessary during an examination with a state in which both eyes are open. The fusion frame chart 153E acts as a fusion stimulation index for aiding the fusion of the left and right eyes to be examined EL and ER.

In this embodiment, the fusion index projecting optical system 132L' is associated with the fixation optical system 132L. When the fusion frame is displayed on the liquid crystal display device 153A, the fusion index indicating optical system 132L' may be integrally formed with the fixation optical system 132L. The fusion index indicating optical system 132L' may be completely separated from the index projecting optical system 132L.

The refractive power measuring optical system 133L includes a measurement light flux projecting optical system 162 for projecting a light flux for objective measurement to the eye to be examined EL and a measurement light flux receiving optical system 163 for receiving reflection light of the projected light flux on the eye to be examined EL.

The measurement light flux projecting optical system 162 includes a measurement light source 164 such as an infrared LED, a collimator lens 165, a cone prism 166, a ring index 167, a relay lens 168, a ring diaphragm 169, a holed prism 170 in which a transmission hole 170a is formed at the center, the dichroic mirrors 161 and 139, the objective lens 138, and the prism P.

The measurement light flux receiving optical system 163 includes the prism P on which reflection light on an eye fundus Ef of the eye to be examined EL is incident, the objective lens 138, the dichroic mirrors 139 and 161, the holed prism 170 having the transmission hole 170a, a reflective mirror 171, a relay lens 172, a movable lens 173, a reflective mirror 174, the dichroic mirror 144, the CCD lens 145, and the CCD 146.

Fig. 14 shows an example of the control system of the optometry apparatus 100. The operation of each part of the optometry apparatus 100 is controlled by a CPU 200. The CPU 200 (control means) executes the control processing of each part of the apparatus based on control programs stored in a ROM 210.

The same association table as that in the first embodiment, which is used to specify the indexes formed on the plurality of index members 153B is stored in the ROM 210.

A drive mechanism portion 300 moves the index members 153B to insert or remove the index members into or from the optical path and includes a stepping motor and a clutch mechanism. When the turret plate is used for the index members 153B, the drive mechanism portion 300 includes a stepping motor for rotating the turret plate by a target angle.

An operating unit 220 is an input device operated by the operator to input an index to be selected and a response regarding the index and includes the lever 106h and the button 106g.

According to the visual acuity chart displaying apparatus 1 having the above-mentioned structure, the optometry measurement can be performed using the index indicated in the following manner.

When the operator operates the operating unit 220 to select an index, the CPU 200 determines the index member 153B on which a target index is formed with reference to the association table.

Next, the CPU 200 controls the drive mechanism portion 300 to move the index member 153B on which the target index is formed to an index indicating position on the optical path.

Then, the CPU 200 turns on the liquid crystal display device 153A to illuminate the target index set in the index indicating position from the rear. Therefore, the target index is projected on the eye to be examined.

According to the optometry apparatus 100 in this embodiment, the flat panel display (liquid crystal display device 153A) is used as the light source for emitting the illumination light for index illumination. Therefore, the apparatus can be reduced in size (thinned) as compared with a conventional case where a light source such as a lamp is used.

Since the index member is illuminated from the rear by the flat panel display having a flat light emitting surface, the index member can be uniformly illuminated, so that it is possible to indicate the index having no unevenness in brightness and contrast.

### <Fourth Embodiment>

Next, an optometry apparatus according to another embodiment of the present invention will be described. The optometry apparatus according to this embodiment has, for example, the same structure as that in the third embodiment except the control system. In the following description, the same reference numerals are provided for the same constituent parts as those in the third embodiment. The contents described in the third embodiment can be arbitrary applied to this embodiment.

Fig. 15 shows an example of a control system of an optometry apparatus 100' according to this embodiment. The CPU 200 includes a mode switching control unit 201 (control means) for switching between the index indicating modes and a display control unit 202 for controlling the liquid crystal display device 153A.

As in the second embodiment, the index indicating modes used for the optometry apparatus 100' include the first index indicating mode for indicating the index of the index member 153B and the second index indicating mode for indicating the index pattern displayed on the liquid crystal display device 153A (third index indicating mode may be further included therein).

It is desirable to selectively employ, for example, a small index as shown in Fig. 7 as the index indicated in the first index indicating mode. It is desirable to selectively employ, for example, a large-size index or a variable index with movement as shown in Fig. 8 as the index indicated in the second index indicating mode. A fixed index such as a landscape chart is also displayed on the liquid crystal display device 153A.

Image data for various index patterns displayed on the liquid crystal display device 153A are stored in the ROM 210.

The mode switching control unit 201 executes the switching between the index indicating modes based on the same control processing as that in the second embodiment.

That is, when it is to be switched to the first index indicating mode, the drive mechanism portion 300 is controlled to move the index member 153B on which the target index is provided to the index indicating position on the optical path. Then, the display control unit 202 is controlled to turn on the liquid crystal display device 153A, thereby illuminating the target index of the index member 153B from the rear.

When it is to be switched to the second index indicating mode, the drive mechanism portion 300 is controlled to retreat the index member 153B from the optical path. Then, the image data for the target index is obtained from the ROM 210. The display control unit 202 is controlled to turn on the liquid crystal display device 153A, thereby displaying the index pattern of the target index.

According to the optometry apparatus 100', the first index indicating mode for indicating the index provided on the index member 153B and the second index indicating mode for indicating the index using the liquid crystal display device 153A can be switched therebetween. Therefore, many different indexes can be indicated using the liquid crystal display device 153A and small indexes can be provided on the index member 153B to use minute indexes.

In the first index indicating mode, the index provided on the index member 153B is subjected to backlight illumination using the liquid crystal display device 153A, so the apparatus can be thinned as compared with the conventional structure having the lamp and the reflective mirror, resulting in that the apparatus can be simplified.

The modified examples described in the second embodiment can be applied to the optometry apparatus 100' according to this embodiment.

The optometry apparatus according to this embodiment is not limited to an optometry apparatus of a type capable of performing the binocular simultaneous objective measurement and the subjective measurement as described in the third and fourth embodiments and thus may be an arbitrary optometry apparatus for indicating an index to the eye to be examined to make a response to a question asking how he/she views the index, thereby executing an eye examination.

## Claims

1. A visual acuity chart displaying apparatus, comprising:
a flat panel display;
an index member including at least one index for eye examination which is formed thereon;
moving means for moving the index member; and
control means for controlling the moving means to move the index member in front of the flat panel display and turning on the flat panel display to illuminate the index member from the rear, to project the index on an eye to be examined.

2. A visual acuity chart displaying apparatus according to claim 1, further comprising display control means for displaying an index pattern for eye to be examined on the flat panel display,
wherein the control means controls the moving means and the display control means to selectively switch between: a first index indicating mode for moving the index member in front of the flat panel display and turning on the flat panel display to illuminate the index member from the rear to project the index on the eye to be examined; and a second index indicating mode for retreating the index member from the position located in front of the flat panel display and displaying the index pattern on the flat panel display to project the index pattern on the eye to be examined.

3. A visual acuity chart displaying apparatus according to claim 2, wherein when the index member on which a plurality of indexes are formed is located in front of the flat panel display in the first index indicating mode, the display control means lights only a region of a display screen of the flat panel display to selectively be illuminated in at least one or more of the plurality of indexes.

4. A visual acuity chart displaying apparatus according to claim 2, wherein the control means performs switching to a third index indicating mode for displaying an index pattern of another index on the flat panel display while the index member is moved in front of the flat panel display and illuminated from the rear to project the index.

5. A visual acuity chart displaying apparatus according to any one of claims 2 to 4, further comprising operating means for selecting one of the index and the index pattern which are projected on the eye to be examined,
wherein the control means performs index indicating mode by switching in accordance with the selected one of the index and the index pattern.

6. An optometry apparatus, comprising:
a flat panel display;
an index member including an index for eye to be examined which is formed thereon;
moving means for moving the index member;
control means for controlling the moving means to move the index member in front of the flat panel display and turning on the flat panel display to illuminate the index member from the rear;
an optical system for projecting the index of the index member illuminated from the rear on an eye to be examined; and
operating means operated to input a response regarding the index projected on the eye to be examined.

7. An optometry apparatus according to claim 6, further comprising display control means for displaying an index pattern for eye to be examined on the flat panel display,
wherein the optical system projects the index pattern displayed on the flat panel display on the eye to be examined,
the operating means is operated to input a response regarding the index pattern projected on the eye to be examined, and
the control means controls the moving means and the display control means to selectively switch between: a first index indicating mode for moving the index member in front of the flat panel display and turning on the flat panel display to illuminate the index member from the rear to project the index on the eye to be examined; and a second index indicating mode for removing the index member from the position located in front of the flat panel display and displaying the index pattern on the flat panel display to project the index pattern on the eye to be examined.
